# FASCICULE DE BREVET EUROPEEN

(11) **EP 3 060 134 B1**
(45) Date de publication et mention de la délivrance du brevet: **11.09.2019**
(21) Numéro de dépôt: 14824051.8
(22) Date de dépôt: 22.10.2014
(51) Int. Cl.: A61B 17/00

(54) **DISPOSITIF DE FIXATION POUR FIXER À UN OS UN TISSU MOU**
FIXIERVORRICHTUNG ZUM FIXIEREN VON WEICHGEWEBE AN EINEM KNOCHEN
FIXATION DEVICE FOR FIXING SOFT TISSUE TO A BONE

(30) Priorité: 25.10.2013 FR 1360475
(43) Date de publication de la demande: 31.08.2016
(73) Titulaire: LCA, 31056 Toulouse Cedex (FR)
(72) Inventeur: POTEL, Jean-François, 31500 Toulouse (FR); JONES, David, 3100 Toulouse (FR); JAVOIS, Christophe, 31120 Goyrans (FR); FRANCESCHI, Jean-Pierre, 13008 Marseille (FR); SBIHI, Abderrahmane, 13002 Marseille (FR); PUJOL, Nicolas, 78150 Le Chesnay (FR); SEIL, Romain, L-5441 Remerschen (LU)
(74) Mandataire: Chevalier, Renaud Philippe
(86) Numéro de dépôt international: PCT/FR2014/052695
(87) Numéro de publication internationale: WO 2015/059415

(56) Documents cités:
- WO-A1-02/11630
- WO-A1-93/12734
- WO-A1-2010/121302
- WO-A2-2012/056384
- US-A1- 2007 049 944

## Description

La présente invention concerne un dispositif de fixation ensemble de fixation pour fixer à un os un tissu mou tel qu'un transplant ligamentaire. Par ailleurs, la présente invention concerne un ensemble de fixation comprenant de tels dispositifs de fixation.

La présente invention trouve notamment application dans le domaine de l'orthopédie et de la chirurgie orthopédique, en particulier pour la reconstruction des ligaments ou des tendons. Plus particulièrement, la présente invention peut être appliquée à la reconstruction d'un ligament croisé ou d'un tendon.

EP2191791A2 décrit un dispositif de fixation comprenant une platine pour fixer un tissu mou, à savoir un greffon de ligament croisé antérieur, à la corticale d'un fémur.

Cependant, le dispositif de fixation de EP2191791A2 ne permet pas au chirurgien de fixer rapidement le tissu mou à l'os. En outre, le dispositif de fixation de EP2191791A2 ne permet pas au chirurgien d'ajuster précisément l'effort de traction exercé sur le tissu mou.

Un dispositif selon le préambule de la revendication 1, est connu du document US-A-2007/0049944.

La présente invention vise notamment à résoudre, en tout ou partie, les problèmes mentionnés ci-avant.

A cet effet, l'invention a pour objet un dispositif de fixation, pour fixer à un os un tissu mou tel qu'un transplant ligamentaire, le dispositif de fixation comprenant :
- au moins un organe d'ancrage configuré pour être ancré dans un trou percé dans l'os, ledit au moins un organe d'ancrage délimitant une cavité configurée déboucher d'une part sur la portion externe du trou et d'autre part sur la portion interne du trou, ledit au moins un organe d'ancrage comprenant des moyens primaires d'encliquetage ;
- un élément d'accrochage configuré pour accrocher le tissu mou ;
- un élément de traction conformé pour une liaison à un fil de traction, l'élément de traction étant conformé pour pénétrer dans la cavité suivant une direction de traction, l'élément de traction étant lié à l'élément d'accrochage de sorte qu'une traction sur l'élément de traction déplace l'élément d'accrochage ; et
- des moyens secondaires d'encliquetage liés à l'élément d'accrochage, les moyens secondaires d'encliquetage étant conformés pour pénétrer dans la cavité, les moyens secondaires d'encliquetage et les moyens primaires d'encliquetage étant configurés pour coopérer mutuellement par déformation élastique de façon à immobiliser l'élément d'accrochage par rapport à l'organe d'ancrage sélectivement suivant au moins deux, de préférence suivant au moins trois, positions d'encliquetage espacées suivant la direction de traction.

En d'autres termes, le dispositif de fixation comprend deux sous-ensembles, à savoir l'organe d'ancrage et un organe encliquetable remplissant les fonctions de traction et d'accrochage du tissu mou. Ainsi, le dispositif de fixation peut être logé complètement dans le trou, sans dépasser partiellement hors du trou.

Ainsi, un tel dispositif de fixation permet de fixer rapidement un tissu mou, tel que ligament ou tendon, à un os en réglant l'effort de traction de manière extemporanée.

Selon un mode de réalisation de l'invention, l'organe d'ancrage a globalement la forme d'une douille, la cavité ayant globalement la forme d'un cylindre, de préférence à base circulaire.

Ainsi, l'organe d'ancrage en forme de douille entoure complètement la cavité.

Selon un mode de réalisation de l'invention, l'organe d'ancrage a une surface externe globalement cylindrique, de préférence à base circulaire, pourvue de reliefs conformés pour s'ancrer dans l'os, les reliefs étant de préférence en forme de filet de vis, en forme de crantage ou en forme d'ergots.

Ainsi, un tel organe d'ancrage peut être logé dans un alésage cylindrique.

Selon un mode de réalisation de l'invention, les moyens primaires d'encliquetage sont formés par au moins deux pattes élastiquement déformables disposées à une extrémité de l'organe d'ancrage, les pattes étant inclinées sur la direction de traction de sorte que la cavité se rétrécit vers la portion interne du trou, les pattes délimitant de préférence une portion de cavité tronconique.

Ainsi, la résistance à la traction augmente progressivement le long de la portion globalement tronconique, ce qui informe le chirurgien de l'approche d'une position d'encliquetage.

Selon une variante de l'invention, les moyens primaires d'encliquetage sont formés par au moins un ergot formant saillie dans la cavité, l'ergot ayant de préférence une forme globalement annulaire. Ainsi, comme chaque ergot est logé dans la cavité, le dispositif de fixation est relativement compact dans la direction de traction.

Selon une variante de l'invention, l'organe d'ancrage a des dimensions transversales inférieures à 8 mm, de préférence inférieure à 6 mm, et une longueur inférieure à 14 mm, de préférence inférieure à 12 mm. Ainsi, de telles dimensions transversales permettent une fixation dans la plupart des grands os, avec un ancrage résistant.

Selon un mode de réalisation de l'invention, deux positions d'encliquetage successives sont espacées d'une distance comprise entre 1 mm et 4 mm, de préférence entre 2 mm et 3 mm, suivant la direction de traction.

Ainsi, de telles distances permettent un réglage fin de l'effort de traction.

Selon un mode de réalisation de l'invention, l'élément d'accrochage comprend une boucle conformée pour le passage du tissu mou, la boucle étant dimensionnée de façon à permettre le passage glissant d'un cylindre de 8 mm de diamètre et le passage serré d'un cylindre de 10 mm de diamètre.

Ainsi, une telle boucle permet d'accrocher aisément le tissu mou.

Selon un mode de réalisation de l'invention, l'élément de traction comprend une broche à chas pour passer un fil de traction, le chas ayant de préférence un diamètre inférieur à 4 mm, de préférence inférieur à 3 mm, et dans lequel l'élément de traction comprend une extrémité de guidage formée par un rétrécissement de section.

Ainsi, l'opérateur peut aisément viser et insérer l'élément de guidage dans la cavité.

Selon un mode de réalisation de l'invention, le dispositif de fixation comprend en outre un instrument de mesure pour mesurer l'effort de traction exercé sur l'élément de traction, tel qu'un dynamomètre, l'instrument de mesure ayant une étendue de mesure inférieure de préférence à 2000 N, de préférence encore à 1000 N.

Ainsi, un tel instrument de mesure permet de mesurer l'effort de traction au cours de son réglage.

Selon un mode de réalisation de l'invention, les moyens primaires d'encliquetage comprennent une seule zone d'encliquetage, les moyens secondaires d'encliquetage comprennent deux zones d'encliquetage de façon à définir les deux positions d'encliquetage distantes.

Ainsi, les moyens primaires d'encliquetage peuvent être relativement compacts.

Selon un mode de réalisation de l'invention, les moyens secondaires d'encliquetage comprennent une seule zone d'encliquetage, les moyens primaires d'encliquetage comprennent deux zones d'encliquetage de façon à définir les deux positions d'encliquetage distantes. Ainsi, les moyens secondaires d'encliquetage sont plus compacts.

Selon un mode de réalisation de l'invention, l'élément d'accrochage, l'élément de traction et les moyens secondaires d'encliquetage sont solidaires, les moyens secondaires d'encliquetage comprenant une tige, dont une portion d'extrémité comporte l'élément d'accrochage et dont la portion d'extrémité opposée comporte l'élément de traction.

Ainsi, le dispositif de fixation comprend seulement deux pièces, ce qui facilite son implantation.

Selon un mode de réalisation de l'invention, les moyens secondaires d'encliquetage comprennent, pour chacune des positions d'encliquetage distantes, une protubérance, par exemple de forme annulaire, formant saillie autour de la tige.

Ainsi, une telle protubérance peut coopérer avec les moyens primaires d'encliquetage. La forme annulaire permet l'encliquetage suivant n'importe quel angle.

Selon une variante de l'invention, chaque protubérance a une forme globalement toroïdale. Ainsi, une telle protubérance permet de répartir uniformément les contraintes.

Selon un mode de réalisation de l'invention, l'élément de traction et la tige sont monoblocs et composés en matériau polymère, de préférence en un polyétherétherkétone (PEEK).

Ainsi, de tels matériaux permettent de réaliser des pièces légères et biocompatibles, radiotransparentes et facilement usinables lorsqu'une reprise est nécessaire.

Selon une variante de l'invention, la tige et l'élément d'accrochage sont percés de façon à permettre le passage d'un fil de traction. Ainsi, un fil de traction peut être maintenu fermement par le dispositif de fixation.

Selon un mode de réalisation de l'invention, le dispositif de fixation comprend en outre une enveloppe conformée pour entourer l'élément d'accrochage, l'élément de traction et les moyens secondaires d'encliquetage, l'enveloppe étant composée de matériau biocompatible et bioassimilable, l'enveloppe étant de préférence tressée et composée d'un matériau polymère sélectionné dans le groupe constitué d'un polyester, d'un polypropylène, d'un polyéthylène haute densité et d'un polyamide.

Ainsi, une telle enveloppe permet d'augmenter la cohésion du tissu mou et de l'os, car elle génère d'importants frottements entre les moyens primaires d'encliquetage et les moyens secondaires d'encliquetage.

Les modes de réalisation et les variantes mentionnés ci-avant peuvent être pris isolément ou selon toute combinaison techniquement admissible.

La présente invention sera bien comprise et ses avantages ressortiront aussi à la lumière de la description qui va suivre, donnée uniquement à titre d'exemple non limitatif et faite en référence aux dessins annexés, dans lesquels :
- la figure 1 est une vue en perspective éclatée et partiellement tronquée d'un dispositif de fixation conforme à un premier mode de réalisation de l'invention ;
- la figure 2 est une vue en perspective, suivant un angle différent de la figure 1, d'une partie du dispositif de fixation de la figure 1 ;
- la figure 3 est une vue similaire à la figure 1, avant son assemblage ;
- la figure 4 est une vue similaire à la figure 1, au cours d'une étape initiale de son assemblage ;
- la figure 5 est une vue similaire à la figure 1, dans une première position de fixation ;
- la figure 6 est une vue de face d'un dispositif de fixation conforme à un deuxième mode de réalisation de l'invention ; et
- la figure 7 est une vue schématique d'un ensemble de fixation comprenant deux dispositifs de fixation conformes au premier mode de réalisation de l'invention.

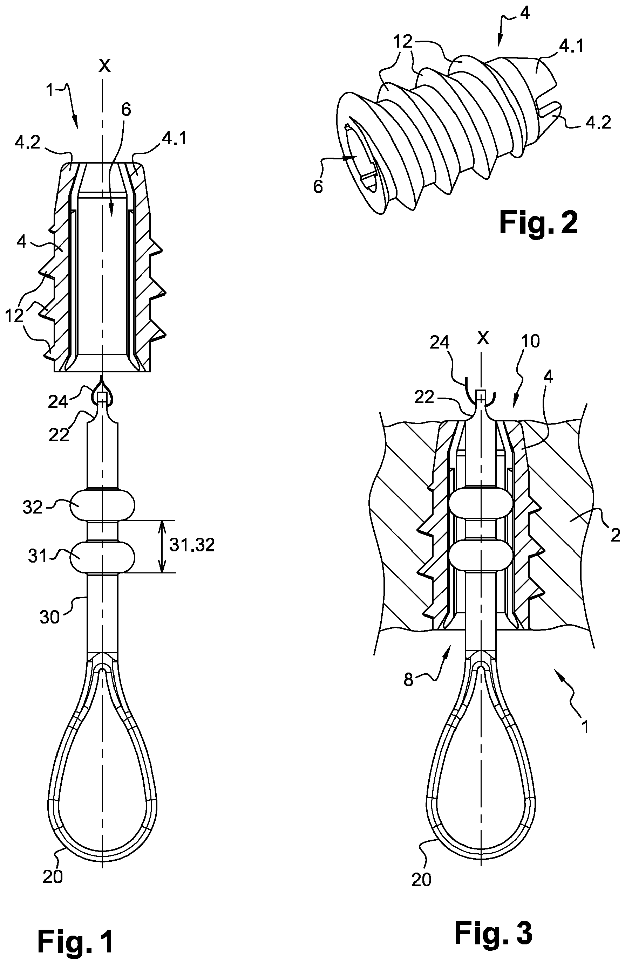

Les figures 1, 2, 3, 4 et 5 illustrent un dispositif de fixation 1, pour fixer à un os 2 un tissu mou non représenté. Le tissu mou peut être par exemple un transplant ligamentaire pour la reconstruction d'un ligament croisé ou d'un tendon.

Le dispositif de fixation 1 comprend un organe d'ancrage 4 qui est configuré pour être ancré dans un trou percé dans l'os 2. L'organe d'ancrage 4 délimite une cavité 6 qui débouche, lorsque le dispositif de fixation est implanté sur l'os 2, d'une part sur la portion externe 8 du trou et d'autre part sur la portion interne 10 du trou. La cavité 6 débouche à ses deux extrémités. Les extrémités de la cavité 6 forment ainsi un premier orifice de passage et un deuxième orifice de passage.

Comme le montre la figure 2, l'organe d'ancrage 4 a globalement la forme d'une douille. La cavité 6 a globalement la forme d'un cylindre à base circulaire. La cavité 6 débouche aux deux extrémités du cylindre circulaire. L'organe d'ancrage 4 a une surface externe globalement cylindrique à base circulaire et coaxiale à la cavité 6. L'organe d'ancrage 4 a ici un diamètre, dimension transversale, d'environ 5 mm.

La surface externe de l'organe d'ancrage 4 est pourvue de reliefs 12 conformés pour s'ancrer dans l'os 2. Dans l'exemple des figures 1 à 5, les reliefs 12 sont en forme de filet de vis.

Le dispositif de fixation 1 comprend en outre un élément d'accrochage 20 qui est configuré pour accrocher le tissu mou. Dans l'exemple des figures, l'élément d'accrochage 20 comprend une boucle qui est conformée pour le passage du tissu mou. La boucle est ici dimensionnée pour permettre le passage glissant d'un cylindre de 8 mm de diamètre et le passage serré d'un cylindre de 10 mm de diamètre.

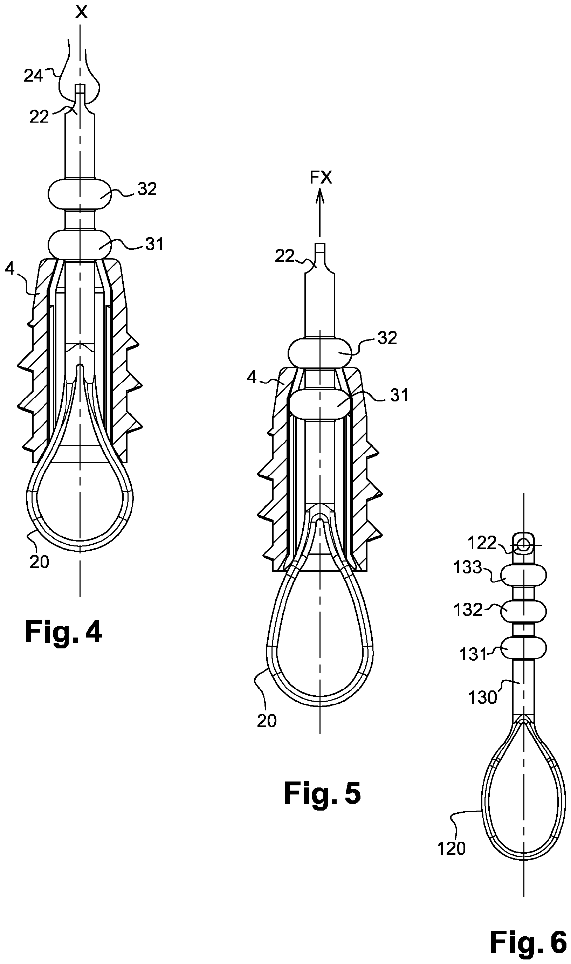

De plus, le dispositif de fixation 1 comprend un élément de traction 22 qui est conformé pour une liaison à un fil de traction 24. Dans l'exemple des figures, l'élément de traction 22 comprend une broche à chas pour passer le fil de traction 24. Le chas a ici un diamètre d'environ 2 mm ; un tel chas est visible sur le mode de réalisation de la figure 6.

L'élément de traction 22 est conformé pour pénétrer dans la cavité 6 suivant une direction de traction X. À cet effet, l'élément de traction 22 comprend une extrémité de guidage formée par un rétrécissement de section.

Par ailleurs, l'élément de traction 22 est lié à l'élément d'accrochage 20 de sorte qu'une traction FX (figure 5) sur l'élément de traction 22 déplace l'élément d'accrochage 20. En l'occurrence, l'élément d'accrochage 20 et l'élément de traction 22 sont solidaires et monoblocs, c'est-à-dire venus de matière.

Par ailleurs, l'organe d'ancrage 4 comprend des moyens primaires d'encliquetage. Les moyens primaires d'encliquetage sont ici formés par deux pattes 4.1 et 4.2 qui sont élastiquement déformables. Les pattes 4.1 et 4.2 sont disposées à une extrémité de l'organe d'ancrage 4. Les pattes 4.1 et 4.2 sont inclinées sur la direction de traction X de sorte que la cavité 6 se rétrécit vers la portion interne 10 du trou. Les pattes 4.1 et 4.2 délimitent ici une portion de cavité tronconique.

En outre, le dispositif de fixation 1 comprend des moyens secondaires d'encliquetage qui sont liés à l'élément d'accrochage 20. Dans l'exemple des figures, les moyens secondaires d'encliquetage comprennent une tige 30, dont une portion d'extrémité comporte l'élément d'accrochage 20 et dont la portion d'extrémité opposée comporte l'élément de traction 22.

Les moyens secondaires d'encliquetage, ici la tige 30, comprennent une seule zone d'encliquetage, alors que les moyens primaires d'encliquetage, ici les pattes 4.1 et 4.2, comprennent deux zones d'encliquetage de façon à définir les deux positions d'encliquetage.

De plus, les moyens secondaires d'encliquetage comprennent, pour chacune des positions d'encliquetage distantes (figure 4 et figure 5), une protubérance annulaire, respectivement 31 et 32, qui forme saillie autour de la tige 30. Chaque protubérance annulaire 31 ou 32 a une forme globalement toroïdale et coaxiale à la direction de traction X.

Dans l'exemple des figures, l'élément d'accrochage 20, l'élément de traction 22 et les moyens secondaires d'encliquetage, c'est-à-dire la tige 30 et les protubérances annulaires 31 et 32 sont solidaires et monoblocs. Ici, l'élément d'accrochage 20, l'élément de traction 22 et la tige 30 sont composés d'un polyétherétherkétone (PEEK).

Les moyens secondaires d'encliquetage sont conformés pour pénétrer dans la cavité 6. Les moyens secondaires d'encliquetage et les moyens primaires d'encliquetage sont configurés pour coopérer mutuellement par déformation élastique de façon à immobiliser l'élément d'accrochage 20 par rapport à l'organe d'ancrage 4, sélectivement suivant deux positions d'encliquetage (figure 4 et figure 5) qui sont espacées suivant la direction de traction X.

Dans l'exemple des figures, les moyens primaires d'encliquetage comprennent une seule zone d'encliquetage, au bout des pattes 4.1 et 4.2, alors que les moyens secondaires d'encliquetage comprennent deux zones d'encliquetage (31 et 32) de façon à définir les deux positions d'encliquetage distantes. Les deux positions d'encliquetage sont ici espacées d'une distance 31.32 d'environ 2,5 mm suivant la direction de traction X.

En d'autres termes, le dispositif de fixation 1 comprend deux sous-ensembles, à savoir l'organe d'ancrage 4, d'une part, et un organe encliquetable remplissant les fonctions de traction et d'accrochage du tissu mou.

Le dispositif de fixation 1 peut en outre comprendre un dynamomètre non représenté pour mesurer l'effort de traction FX (figure 5) exercé sur l'élément de traction 22.

De plus, le dispositif de fixation 1 comprend une enveloppe non représentée qui est conformée pour entourer l'élément d'accrochage 20, l'élément de traction 22 et les moyens secondaires d'encliquetage (30, 31 et 32). L'enveloppe est composée de matériau biocompatible et bioassimilable, ici un polyester.

En implantation, l'opérateur insère l'élément de traction 22 dans la cavité 6 avec son fil de traction 24. Puis, l'opérateur exerce un effort de traction FX sur le fil de traction 24, de façon à passer les moyens secondaires d'encliquetage dans la cavité 6. Ensuite, la protubérance annulaire 31 déforme élastiquement les pattes 4.1 et 4.2, puis sort de la cavité 6. La première position d'encliquetage (figure 5) est atteinte et la protubérance annulaire 31 est retenue contre l'extrémité des pattes 4.1 et 4.2. Si nécessaire, l'opérateur peut tirer sur le fil de traction 24 jusqu'à atteindre la deuxième position (figure 4), dans laquelle la protubérance annulaire 32 est retenue contre l'extrémité des pattes 4.1 et 4.2.

La figure 6 illustre une partie d'un dispositif de fixation 101 conforme à un deuxième mode de réalisation de l'invention. Dans la mesure où le dispositif de fixation 101 est similaire au dispositif de fixation 1, la description du dispositif de fixation 1 donnée ci-avant en relation avec les figures 1 et 2 peut être transposée au dispositif de fixation 101, à l'exception des différences notables énoncées ci-après.

Un composant du dispositif de fixation 101 identique ou correspondant, par sa structure ou par sa fonction, à un composant du dispositif de fixation 1 porte la même référence numérique augmentée de 100. On définit ainsi un élément d'accrochage 120, un élément de traction 122, ainsi qu'une tige 130 et des protubérances annulaires 131 et 132 qui forment des moyens secondaires d'encliquetage.

Le dispositif de fixation 101 diffère du dispositif de fixation 1, car il comprend trois protubérances annulaires 131, 132 et 133, alors le que dispositif de fixation 1 ne comprend que deux protubérances annulaires 131 et 132. Les trois protubérances annulaires 131, 132 et 133 offrent une plage d'ajustement plus étendue.

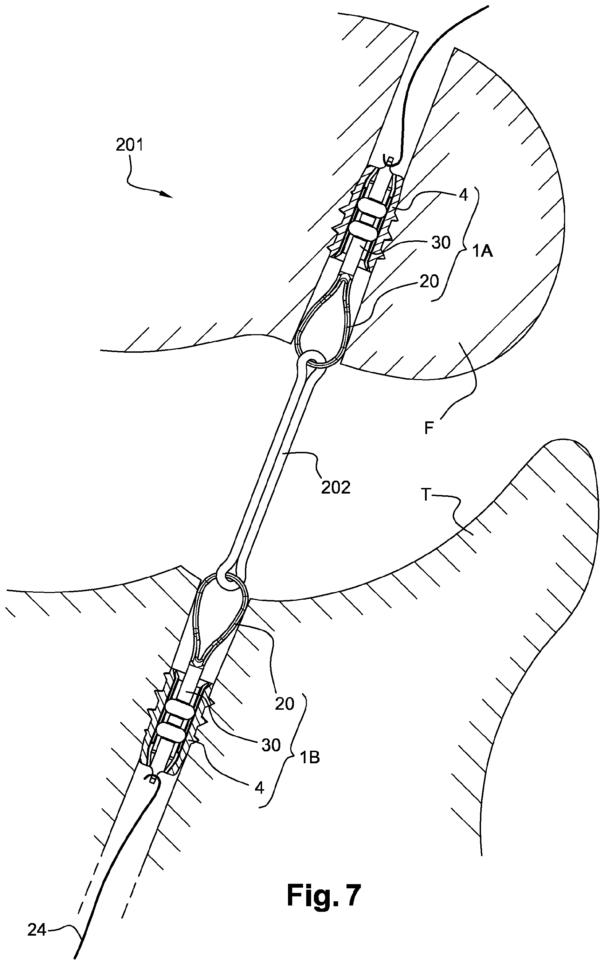

En service, la figure 7 illustre deux dispositifs de fixation 1A et 1B qui sont semblables au dispositif de fixation 1 décrit ci-avant en relation avec les figures 1 à 5. Les dispositifs de fixation 1A et 1B composent un ensemble de fixation 201.

Le dispositif de fixation 1A est fixé dans un tunnel percé dans le fémur et le dispositif de fixation 1B est fixé dans un tunnel percé dans le tibia. Les dispositifs de fixation 1A et 1B sont guidés dans ces tunnels au moyen de fils de traction 24. Un transplant de ligament croisé 202, tissu mou, est accroché, par ses deux extrémités, respectivement aux dispositifs de fixation 1A et 1B. Ainsi, l'ensemble de fixation 201 et le transplant de ligament croisé 202 permettent au ligament de retrouver une fonctionnalité normale.

Bien entendu, la présente invention n'est pas limitée aux modes de réalisation particuliers décrits dans la présente demande de brevet, ni à des modes de réalisation à la portée de l'homme du métier. D'autres modes de réalisation peuvent être envisagés sans sortir du cadre de l'invention, à partir de tout élément équivalent à un élément indiqué dans la présente demande de brevet.

## Revendications

1. Dispositif de fixation (1 ; 101), pour fixer à un os (2) un tissu mou tel qu'un transplant ligamentaire, le dispositif de fixation (1 ; 101) comprenant :
- au moins un organe d'ancrage (4) configuré pour être ancré dans un trou percé dans l'os (2), ledit au moins un organe d'ancrage (4) délimitant une cavité (6) configurée pour déboucher d'une part sur la portion externe (8) du trou et d'autre part sur la portion interne (10) du trou, ledit au moins un organe d'ancrage (4) comprenant des moyens primaires d'encliquetage ;
- un élément d'accrochage (20; 120) configuré pour accrocher le tissu mou ;
- un élément de traction (22 ; 122) conformé pour une liaison à un fil de traction (24), l'élément de traction (22 ; 122) étant conformé pour pénétrer dans la cavité (6) suivant une direction de traction (X), l'élément de traction (22 ; 122) étant lié à l'élément d'accrochage (20 ; 120) de sorte qu'un effort de traction sur l'élément de traction (22; 122) déplace l'élément d'accrochage (20 ; 120) ; et
- **caractérisé en ce que** le dispositif est pourvu de moyens secondaires d'encliquetage liés à l'élément d'accrochage (20; 120), les moyens secondaires d'encliquetage étant conformés pour pénétrer dans la cavité (6), les moyens secondaires d'encliquetage et les moyens primaires d'encliquetage étant configurés pour coopérer mutuellement par déformation élastique de façon à immobiliser l'élément d'accrochage (20; 120) par rapport à l'organe d'ancrage (4) sélectivement suivant au moins deux, de préférence suivant au moins trois, positions d'encliquetage espacées suivant la direction de traction (X).

2. Dispositif de fixation (1 ; 101) selon la revendication 1, dans lequel l'organe d'ancrage (4) a globalement la forme d'une douille, la cavité (6) ayant globalement la forme d'un cylindre, de préférence à base circulaire.

3. Dispositif de fixation (1) selon l'une des revendications précédentes, dans lequel l'organe d'ancrage (4) a une surface externe globalement cylindrique, de préférence à base circulaire, pourvue de reliefs (12) conformés pour s'ancrer dans l'os (2), les reliefs étant de préférence en forme de filet de vis, en forme de crantage ou en forme d'ergots.

4. Dispositif de fixation (1) selon l'une des revendications précédentes, dans lequel les moyens primaires d'encliquetage sont formés par au moins deux pattes (4.1, 4.2) élastiquement déformables disposées à une extrémité de l'organe d'ancrage (4), les pattes (4.1, 4.2) étant inclinées sur la direction de traction (X) de sorte que la cavité (6) se rétrécit vers la portion interne (10) du trou, les pattes (4.1, 4.2) délimitant de préférence une portion de cavité tronconique.

5. Dispositif de fixation (1) selon l'une des revendications précédentes, dans lequel deux positions d'encliquetage successives sont espacées d'une distance (31.32) comprise entre 1 mm et 4 mm, de préférence entre 2 mm et 3 mm, suivant la direction de traction (X).

6. Dispositif de fixation (1) selon l'une des revendications précédentes, dans lequel l'élément d'accrochage (20; 120) comprend une boucle conformée pour le passage du tissu mou, la boucle étant dimensionnée de façon à permettre le passage glissant d'un cylindre de 8 mm de diamètre et le passage serré d'un cylindre de 10 mm de diamètre.

7. Dispositif de fixation (1) selon l'une des revendications précédentes, dans lequel l'élément de traction (22 ; 122) comprend une broche à chas pour passer un fil de traction (24), le chas ayant de préférence un diamètre inférieur à 4 mm, de préférence inférieur à 3 mm, et dans lequel l'élément de traction (22 ; 122) comprend une extrémité de guidage formée par un rétrécissement de section.

8. Dispositif de fixation (1) selon l'une des revendications précédentes, comprenant en outre un instrument de mesure pour mesurer l'effort de traction exercé sur l'élément de traction, tel qu'un dynamomètre, l'instrument de mesure ayant une étendue de mesure inférieure de préférence à 2000 N, de préférence encore à 1000 N.

9. Dispositif de fixation (1) selon l'une des revendications précédentes, dans lequel les moyens primaires d'encliquetage comprennent une seule zone d'encliquetage, les moyens secondaires d'encliquetage comprennent deux zones d'encliquetage de façon à définir les deux positions d'encliquetage distantes.

10. Dispositif de fixation (1; 101) selon l'une des revendications précédentes, dans lequel l'élément d'accrochage (20; 120), l'élément de traction (22 ; 122) et les moyens secondaires d'encliquetage sont solidaires, les moyens secondaires d'encliquetage comprenant une tige (30 ; 130), dont une portion d'extrémité comporte l'élément d'accrochage (20 ; 120) et dont la portion d'extrémité opposée comporte l'élément de traction (22 ; 122).

11. Dispositif de fixation (1 ; 101) selon la revendication 10, dans lequel les moyens secondaires d'encliquetage comprennent, pour chacune des positions d'encliquetage distantes, une protubérance (31, 32 ; 131, 132, 133), par exemple de forme annulaire, formant saillie autour de la tige (30 ; 130).

12. Dispositif de fixation (1; 101) selon l'une des revendications 10 à 11, dans lequel l'élément de traction (22 ; 122) et la tige (30 ; 130) sont monoblocs et composés en matériau polymère, de préférence en un polyétherétherkétone (PEEK).

13. Dispositif de fixation (1) selon l'une des revendications précédentes, comprenant en outre une enveloppe conformée pour entourer l'élément d'accrochage (20), l'élément de traction (22) et les moyens secondaires d'encliquetage, l'enveloppe étant composée de matériau biocompatible et bioassimilable, l'enveloppe étant de préférence tressée et composée d'un matériau polymère sélectionné dans le groupe constitué d'un polyester, d'un polypropylène, d'un polyéthylène haute densité et d'un polyamide.

14. Dispositif de fixation (1) selon l'une des revendications précédentes, dans lequel les moyens secondaires d'encliquetage comprennent une seule zone d'encliquetage, les moyens primaires d'encliquetage comprennent deux zones d'encliquetage de façon à définir les deux positions d'encliquetage.

## Patentansprüche

1. Fixiervorrichtung (1; 101), um ein Weichgewebe, wie etwa ein Bandtransplantat, an einem Knochen (2) zu fixieren, wobei die Fixiervorrichtung (1; 101) umfasst:
- mindestens ein Verankerungsglied (4), das dafür konfiguriert ist, in einem Loch verankert zu werden, das in den Knochen (2) gebohrt ist, wobei das mindestens eine Verankerungsglied (4) einen Hohlraum (6) begrenzt, der dafür konfiguriert ist, einerseits am äußeren Abschnitt (8) des Lochs, und andererseits am inneren Abschnitt (10) des Lochs zu münden, wobei das mindestens eine Verankerungsglied (4) primäre Verrastungsmittel umfasst;
- ein Einhängelement (20; 120), das dafür konfiguriert ist, das Weichgewebe einzuhängen;
- ein Zugelement (22; 122), das für eine Verbindung mit einem Zugdraht (24) ausgestaltet ist, wobei das Zugelement (22; 122) dafür ausgestaltet ist, entlang einer Zugrichtung (X) in den Hohlraum (6) einzudringen, wobei das Zugelement (22; 122) derart mit dem Einhängelement (20; 120) verbunden ist, dass eine Zugkraft auf das Zugelement (22; 122) das Einhängelement (20; 120) bewegt; und
- **dadurch gekennzeichnet, dass** die Vorrichtung mit sekundären Verrastungsmitteln versehen ist, die mit dem Einhängelement (20; 120) verbunden sind, wobei die sekundären Verrastungsmittel dafür ausgestaltet sind, in den Hohlraum (6) einzudringen, wobei die sekundären Verrastungsmittel und die primären Verrastungsmittel dafür konfiguriert sind, durch elastische Verformung so miteinander zusammenzuwirken, dass sie das Einhängelement (20; 120) in Bezug auf das Verankerungsglied (4) selektiv entlang mindestens zwei, vorzugsweise entlang mindestens drei entlang der Zugrichtung (X) beabstandeten Verrastungspositionen immobilisieren.

2. Fixiervorrichtung (1; 101) nach Anspruch 1, wobei das Verankerungsglied (4) allgemein die Form einer Hülse aufweist, wobei der Hohlraum (6) allgemein die Form eines Zylinders, vorzugsweise mit kreisrunder Basis, aufweist.

3. Fixiervorrichtung (1) nach einem der vorstehenden Ansprüche, wobei das Verankerungsglied (4) eine allgemein zylinderförmige Außenfläche, vorzugsweise mit kreisrunder Basis, aufweist, die mit Erhebungen (12) versehen ist, welche dafür ausgestaltet sind, sich im Knochen (2) zu verankern, wobei die Erhebungen vorzugsweise in Schraubengewindeform, in Zahnform oder in Zapfenform vorliegen.

4. Fixiervorrichtung (1) nach einem der vorstehenden Ansprüche, wobei die primären Verrastungsmittel von mindestens zwei elastisch verformbaren Klauen (4.1, 4.2) gebildet werden, die an einem Ende des Verankerungsglieds (4) angeordnet sind, wobei die Klauen (4.1, 4.2) derart zur Zugrichtung (X) geneigt sind, dass sich der Hohlraum (6) zum inneren Abschnitt (10) des Loches hin verengt, wobei die Klauen (4.1, 4.2) vorzugsweise einen kegelstumpfförmigen Hohlraumabschnitt begrenzen.

5. Fixiervorrichtung (1) nach einem der vorstehenden Ansprüche, wobei zwei aufeinanderfolgende Verrastungspositionen entlang der Zugrichtung (X) um eine Entfernung (31.32) im Bereich zwischen 1 mm und 4 mm, vorzugweise zwischen 2 mm und 3 mm beabstandet sind.

6. Fixiervorrichtung (1) nach einem der vorstehenden Ansprüche, wobei das Einhängelement (20; 120) eine Schlinge umfasst, die für das Durchziehen des Weichgewebes ausgestaltet ist, wobei die Schlinge so bemessen ist, dass sie das gleitende Durchziehen eines Zylinders von 8 mm Durchmesser, und das straffe Durchziehen eines Zylinders von 10 mm Durchmesser ermöglicht.

7. Fixiervorrichtung (1) nach einem der vorstehenden Ansprüche, wobei das Zugelement (22; 122) eine Spindel mit Öhr umfasst, um einen Zugdraht (24) durchzuziehen, wobei das Öhr vorzugsweise einen Durchmesser von weniger als 4 mm, vorzugsweise weniger als 3 mm aufweist, und wobei das Zugelement (22; 122) ein Führungsende umfasst, das von einer Querschnittsverengung gebildet wird.

8. Fixiervorrichtung (1) nach einem der vorstehenden Ansprüche, die weiter ein Messinstrument wie etwa ein Dynamometer umfasst, um die Zugkraft, die auf das Zugelement ausgeübt wird, zu messen, wobei das Messinstrument einen Messbereich vorzugsweise von weniger als 2000 N, noch bevorzugter als 1000 N aufweist.

9. Fixiervorrichtung (1) nach einem der vorstehenden Ansprüche, wobei die primären Verrastungsmittel eine einzige Verrastungszone umfassen, die sekundären Verrastungsmittel zwei Verrastungszonen umfassen, sodass sie die zwei entfernten Verrastungspositionen definieren.

10. Fixiervorrichtung (1; 101) nach einem der vorstehenden Ansprüche, wobei das Einhängelement (20; 120), das Zugelement (22; 122) und die sekundären Verrastungsmittel fest miteinander verbunden sind, wobei die sekundären Verrastungsmittel einen Stab (30; 130) umfassen, von dem ein Endabschnitt das Einhängelement (20; 120) umfasst, und von dem der gegenüberliegende Endabschnitt das Zugelement (22; 122) umfasst.

11. Fixiervorrichtung (1; 101) nach Anspruch 10, wobei die sekundären Verrastungsmittel für jede der entfernten Verrastungspositionen eine zum Beispiel ringförmige Ausbauchung (31, 32; 131, 132, 133) umfassen, die einen Vorsprung um den Stab (30; 130) herum bildet.

12. Fixiervorrichtung (1; 101) nach einem der Ansprüche 10 bis 11, wobei das Zugelement (22; 122) und der Stab (30; 130) einstückig sind und aus Polymermaterial, vorzugsweise aus einem Polyetheretherketon (PEEK) bestehen.

13. Fixiervorrichtung (1) nach einem der vorstehenden Ansprüche, die weiter eine Hülle umfasst, die dafür ausgestaltet ist, das Einhängelement (20), das Zugelement (22) und die sekundären Verrastungsmittel zu umgeben, wobei die Hülle aus biokompatiblem und bioassimilierbarem Material besteht, wobei die Hülle vorzugsweise geflochten ist und aus einem Polymermaterial besteht, ausgewählt aus der Gruppe, die von einem Polyester, einem Polypropylen, einem hochdichten Polyethylen und einem Polyamid gebildet wird.

14. Fixiervorrichtung (1) nach einem der vorstehenden Ansprüche, wobei die sekundären Verrastungsmittel eine einzige Verrastungszone umfassen, die primären Verrastungsmittel zwei Verrastungszonen umfassen, sodass sie die zwei Verrastungspositionen definieren.

## Claims

1. A fastening device (1; 101), for fastening a soft tissue such as a ligament transplant to a bone (2), the fastening device (1; 101) comprising:
- at least one anchoring member (4) configured to be anchored in a hole drilled in the bone (2), said at least one anchoring member (4) delimiting a cavity (6) configured to open, on the one hand, onto the outer segment (8) of the hole, and on the other hand, onto the inner segment (10) of the hole, said at least one anchoring member (4) comprising primary snap-fitting means;
- a hooking element (20; 120) configured to hook the soft tissue;
- a pulling element (22; 122) shaped for a link to a pull wire (24), the pulling element (22; 122) being shaped to penetrate into the cavity (6) in a pulling direction (X), the pulling element (22; 122) being linked to the hooking element (20; 120) so that a pulling force on the pulling element (22; 122) moves the hooking element (20; 120); and
- **characterized in that** the device is provided with secondary snap-fitting means linked to the hooking element (20; 120), the secondary snap-fitting means being shaped to penetrate into the cavity (6), the secondary snap-fitting means and the primary snap-fitting means being configured to mutually cooperate by elastic deformation so as to immobilize the hooking element (20; 120) relative to the anchoring member (4) selectively in at least two , preferably in at least three, snap-fitting positions spaced along the pulling direction (X).

2. The fastening device (1; 101) according to claim 1, wherein the anchoring member (4) has generally the shape of a socket, the cavity (6) having generally the shape of a cylinder, preferably with a circular base.

3. The fastening device (1) according to any of the preceding claims, wherein the anchoring member (4) has a generally cylindrical outer surface, preferably with a circular base, provided with reliefs (12) shaped to be anchored in the bone (2), the reliefs being preferably in the shape of a screw thread, in the shape of notches or in the shape of lugs.

4. The fastening device (1) according to any of the preceding claims, wherein the primary snap-fitting means are formed by at least two elastically deformable tabs (4.1, 4.2) disposed at one end of the anchoring member (4), the tabs (4.1, 4.2) being inclined on the pulling direction (X) so that the cavity (6) narrows towards the inner segment (10) of the hole, the tabs (4.1, 4.2) preferably delimiting a frustoconical cavity segment.

5. The fastening device (1) according to any of the preceding claims, wherein two successive snap-fitting positions are spaced by a distance (31.32) comprised between 1 mm and 4 mm, preferably between 2 mm and 3 mm, along the pulling direction (X).

6. The fastening device (1) according to any of the preceding claims, wherein the hooking element (20; 120) comprises a loop shaped for the passage of the soft tissue, the loop being dimensioned so as to allow the sliding passage of a cylinder of 8 mm in diameter and the tight passage of a cylinder of 10 mm in diameter.

7. The fastening device (1) according to any of the preceding claims, wherein the pulling element (22; 122) comprises an eyelet pin for passing a pull wire (24), the eyelet preferably having a diameter less than 4 mm, preferably less than 3 mm, and wherein the pulling element (22; 122) comprises a guide end formed by a sectional narrowing.

8. The fastening device (1) according to any of the preceding claims, further comprising a measuring instrument for measuring the pulling force exerted on the pulling element, such as a dynamometer, the measuring instrument having a measuring range preferably less than 2000 N, more preferably than 1000 N.

9. The fastening device (1) according to any of the preceding claims, wherein the primary snap-fitting means comprise a single snap-fitting area, the secondary snap-fitting means comprise two snap-fitting areas so as to define both distant snap-fitting positions.

10. The fastening device (1, 101) according to any of the preceding claims, wherein the hooking element (20; 120), the pulling element (22; 122) and the secondary snap-fitting means are secured together, the secondary snap-fitting means comprising a rod (30; 130), whose end segment includes the hooking element (20; 120) and whose opposite end segment includes the pulling element (22, 1 22).

11. The fastening device (1, 101) according to claim 10, wherein the secondary snap-fitting means comprise, for each of the distant snap-fitting positions, a protuberance (31, 32; 131, 132, 133), for example of an annular shape, protruding around the rod (30; 130).

12. The fastening device (1,101) according to any of claims 10 to 11, wherein the pulling element (22; 122) and the rod (30; 130) are formed in one piece and composed of a polymer material, preferably of a polyetheretherketone (PEEK).

13. The fastening device (1) according to any of the preceding claims, further comprising an enclosure shaped to surround the hooking element (20), the pulling element (22) and the secondary snap-fitting means, the enclosure being composed of biocompatible and bioassimilable material, the enclosure being preferably braided and composed of a polymer material selected from the group consisting of polyester, polypropylene, high-density polyethylene and polyamide.

14. The fastening device (1) according to any of the preceding claims, wherein the secondary snap-fitting means comprise a single snap-fitting area, the primary snap-fitting means comprise two snap-fitting areas so as to define both snap-fitting positions.
